# EUROPEAN PATENT APPLICATION

(11) **EP 4 455 261 A1**
(43) Date of publication of application: **30.10.2024**
(21) Application number: 22910144.9
(22) Date of filing: 22.12.2022
(51) Int. Cl.: C12M 3/00, C12M 1/38, C12M 1/36, C12M 1/34, C12M 1/04

(54) **INTEGRATED AUTOMATED CELL CULTURE DEVICE**

(30) Priority: 22.12.2021 US 202163292433 P
(71) Applicant: Medigen Biotechnology Corp., Taipei City, 115 (TW)
(72) Inventor: CHANG, Hsun-Liang, Taipei City, Taiwan 115 (TW); LIN, Chieh-Liang, Taipei City, Taiwan 115 (TW); YANG, Chih-Ya, Taipei City, Taiwan 115 (TW); LIU, Chun-Hsien, Taipei City, Taiwan 115 (TW); CHANG, Stanley, Taipei City, Taiwan 115 (TW)
(74) Representative: Vossius & Partner Patentanwälte Rechtsanwälte mbB
(86) International application number: PCT/CN2022/141081
(87) International publication number: WO 2023/116833

(57) **Abstract**

The integrated automated cell culture device includes a main body, a cover, a connection seat, a gas module, a temperature module, at least one slide track, and a clamping member. The main body encloses a culture room for receiving a culture bag. The cover is disposed on the opening of the culture room. The connection seat is received in a receiving slot of the main body and is adapted for a pipe to penetrate. The gas module detects the carbon dioxide's concentration and selectively injects gas into the culture room. The temperature module detects the temperature in the culture room and selectively heats the culture room. The slide track is disposed on the bottom of the culture room. The clamping member is slidably disposed on the slide track and clamps the culture bag to make the fluid in the culture bag move to one side of the culture bag.

## Description

### FIELD OF THE INVENTION

The present disclosure relates to a cell culture device, more especially to an integrated automated cell culture device.

### BACKGROUND OF THE INVENTION

Nowadays, the medical treatments to the cancers not only include traditional chemical medicine, radiation treatment, or removing the cancer cells by surgery, but also include gene therapy or cell therapy to prevent the severe side effects. For example, in the cell therapy, the immune cells of the patient himself may not cause immune rejection so that the cells can kill the cancer cells better than the immune cells provided from others. Also, the therapy can reduce the physical burden on the patient.

To take the cell therapy, the cells of the patient or others have to be cultured in vitro, and the increased cells are further injected into the body of the patient. The cell culture usually undergoes in a soft culture bag. New culture fluid is injected into the culture bag according to the amount of the cells increased. The culture fluid is placed in a laboratory environment with specific temperature and humidity.

Specifically, to acquire how the cells grow, observation to the culture fluid is necessary. Practically, the culture fluid is manually extracted from the culture bag for a small amount several times in order to count the number of the cells. And then, according to the amount of the cells, a corresponding amount of new culture fluid is further injected into the culture bag. In the whole process of cell culture, the step has to be manually undergone several times. Thus, the labor cost is high. In addition, the cells have risks to be contaminated, and the deviation may happen due to the manual process. Thus, the quality of the cells is not stable.

This is also why the cell culture process cannot be automated. Thus, the cost of cell culture is too high for a general patient to afford.

The present invention is, therefore, arisen to obviate or at least mitigate the above mentioned disadvantages.

### SUMMARY OF THE INVENTION

One of the objects of the present invention is to provide an integrated automated cell culture device which is able to monitor and culture the cells of the culture bag in an integrated device. Thereby automated cell culture can be achieved.

One of the objects of the present invention is to provide an integrated automated cell culture device which is easy to monitor the cell density in the culture bag without extracting the culture fluid in the culture bag. Thus, the culture environment can be kept clean, and the cell density can be acquired quickly.

To achieve the above and other objects, the integrated automated cell culture device of the present invention includes a main body, a cover, a connection seat, a gas module, at least one slide track, and a clamping member.

The main body encloses a culture room. The top of the main body is formed with an opening communicating the culture room. The main body further has a receiving groove at a lateral side thereof communicating the culture room. The bottom of the culture room is adapted for a culture bag to rest on. The cover is detachably disposed on the main body to cover the opening in order to selectively close the culture room. The connection seat is adapted for inserted into the receiving groove. The connection seat is formed with at least one through hole. The at least one through hole is adapted for at least one pipeline to insert through so that the at least one pipeline connects the culture room and the exterior. An end of the at least one pipeline toward the culture room is adapted for connecting to the culture bag. The gas module includes a carbon dioxide senser and a gas source connecting member. The carbon dioxide sensor is arranged in the culture room and is electrically connected to the gas source connecting member. The gas source connecting member is disposed on the main body to be adapted for a gas source at the exterior to connect to so as to provide gas into the culture room. The temperature module is disposed on at least one of the main body and the cover, and includes a heater and a temperature detecting member. The temperature detecting member is electrically connected to the heater. The temperature detecting member is adapted for detecting a temperature in the culture room and selectively drives the heater to heat up the culture room. The at least one slide track is arranged in or beside the culture room and extends along a sliding direction. The clamping member is slidably disposed on the at least one slide track and is adapted for pressing the culture bag in order to prohibit a fluid in the culture bag to flow to one side of the culture bag. The clamping member is slidable on the culture bag. Preferably, the device further includes a pulling mechanism disposed at least at end of the culture room in order to clamp an end of the culture bag.

Preferably, the device further includes an illumination counting module. The illumination counting module includes a light source and a light receiver. The light source is disposed on one of the culture room and a face of the cover facing the culture room, and the light receiver is disposed on the other one of the culture room and the face of the cover facing the culture room so that the culture bag is located between the light source and the light receiver when the culture bag is placed in the culture room. The light source is adapted for emitting light having a wavelength of 400-600 nm toward the light receiver. Light emitted by the light source penetrates the culture bag and is further received by the light receiver so as to acquire an optical density (OD) of the fluid in the culture bag. Thereby a cell density in the culture bag is obtained.

In some embodiments, the culture bag has at least one observation portion outward extending horizontally. The fluid and cells in the culture bag are free to enter or leaving the observation portion. Positions of the light source and the light receiver correspond to a position of the observation portion so that the light emitted by the light source penetrates the culture bag via the observation portion.

In some embodiments, the cover is formed with an observation opening at an intermediate portion of the cover. A heating glass is arranged on the observation opening to cover the observation opening. A pivotable cover is arranged at a side of the heating glass opposite to the culture room. The pivotable cover selectively covers the heating glass from outside. The heating glass is said heater. The temperature detecting member is disposed on the face of the cover facing the culture room.

In some embodiments, the gas module and the connection seat are arranged at two opposite ends of the culture room.

In some embodiments, the device further includes a ventilation rack. The ventilation rack is placed on the bottom of the culture room to support the culture bag. Preferably, the ventilation rack is formed with a plurality of hollow portions so as to facilitate the ventilation of the bottom of the culture bag.

In some embodiments, the device further includes a cooling box and a pump. The cooling box is arranged in the main body to receive at least one culture fluid container. The pump is also disposed on the main body. The at least one pipeline is connected to the at least one culture fluid container via the pump so as to provide fluid into the culture bag,

Thereby, the data of the environment inside the culture room can be quickly acquired without interfering the cell culture by human, and the necessary processes can be made manually or automatically. Furthermore, the cell density in the culture bag can be acquired by the illumination counting module so as to observe the growth of cells. Thereby, the information can be a reference to adjust the culture fluid, the gas, the temperature, the humidity, and so on. Thus, automated cell culture which is stable can be achieved.

The present invention will become more obvious from the following description when taken in connection with the accompanying drawings, which show, for purpose of illustrations only, the preferred embodiment(s) in accordance with the present invention.

### BRIEF DESCRIPTION OF THE DRAWINGS

The present disclosure can be more fully understood by reading the following detailed description of the embodiment, with reference made to the accompanying drawings as follows:

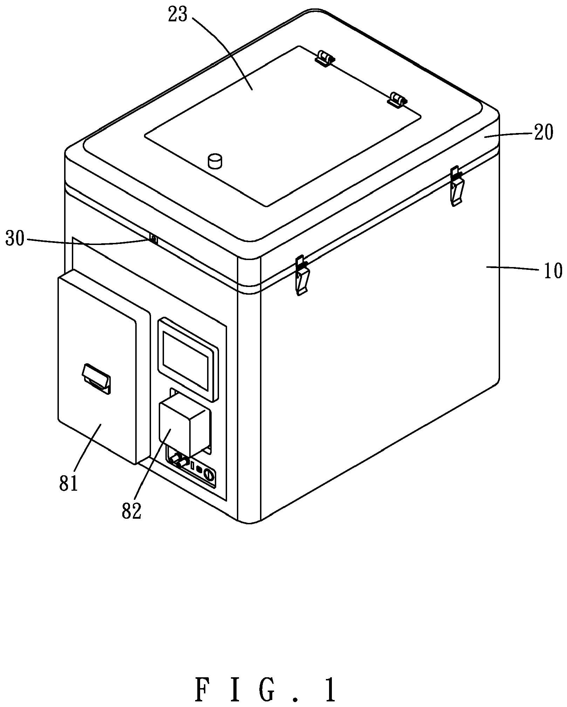

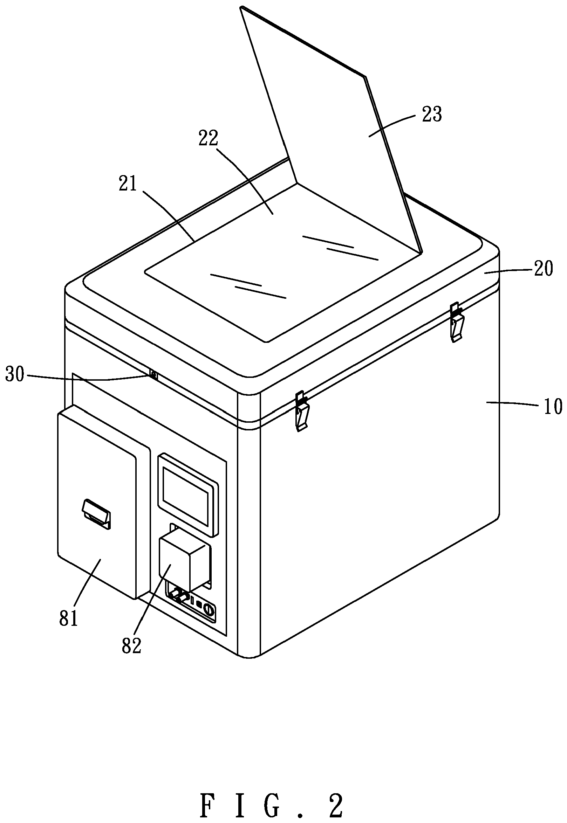

Fig. 1 and Fig. 2 are stereograms showing an integrated automated cell culture device of the present invention;

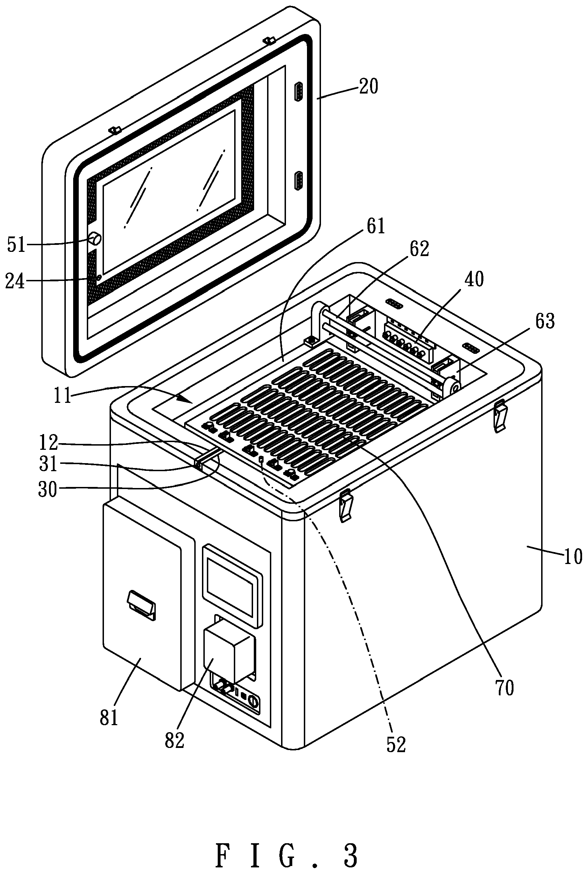

Fig. 3 is a breakdown drawing showing an integrated automated cell culture device of the present invention;

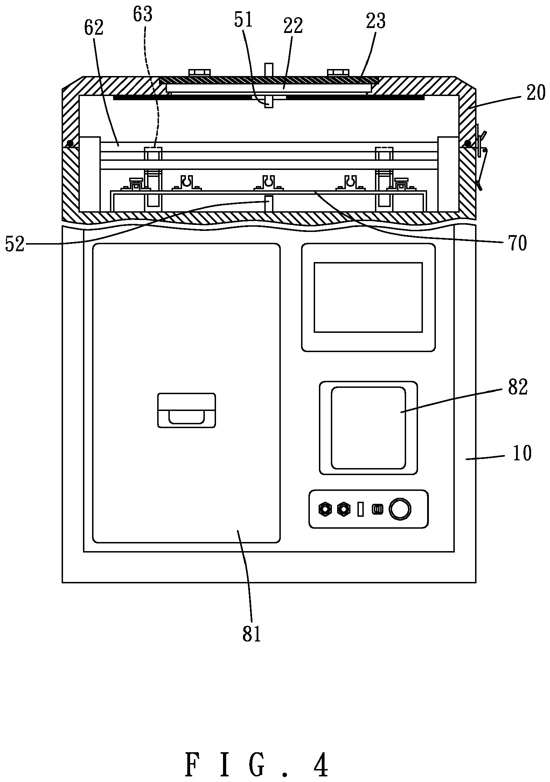

Fig. 4 is a partial section view showing an integrated automated cell culture device of the present invention;

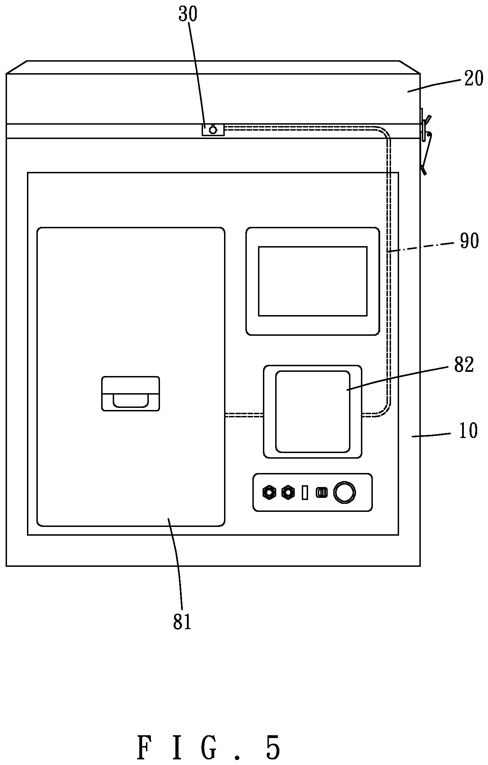

Fig. 5 is a front view showing an integrated automated cell culture device of the present invention;
Fig. 6 is a graph of the wavelength of the light source and the optical density showing a first embodiment of the present invention;
Fig. 7 is a graph of the optical density and the cell density showing a first embodiment of the present invention when the wavelength of the light source is 290 nm;
Fig. 8 is a graph of the wavelength of the light source and the optical density showing a second embodiment of the present invention;
Fig. 9 is a graph of the optical density and the cell density showing a second embodiment of the present invention when the wavelength of the light source is 290 nm;
Fig. 10 is a graph of the wavelength of the light source and the optical density showing a third embodiment of the present invention;
Fig. 11 is a graph of the optical density and the cell density showing a third embodiment of the present invention when the wavelength of the light source is 290 nm;
Fig. 12 is a graph of the optical density and the cell density showing a third embodiment of the present invention when the wavelength of the light source varies.
Fig. 13 is a table of the optical density and the cell density showing a third embodiment of the present invention when the wavelength of the light source varies.

### DETAILED DESCRIPTION OF THE PREFERRED EMBODIMENTS

The present invention provides an integrated automated cell culture device, at least including a main body 10, a cover 20, a connection seat 30, a gas module 40, a temperature module, a culture bag positioning mechanism, and an illumination counting module, preferably further including a ventilation rack 70 and a culture fluid providing mechanism.

The main body 10 is substantially box-shaped and encloses a culture room 11. The top of the main body 10 is formed with an opening communicating the culture room 11. The main body 10 further has a receiving groove 12 communicating the culture room 11 at a lateral side thereof. Preferably, the receiving groove 12 is a notch to be formed beside the opening of the main bod 10. The bottom of the culture room 11 is adapted for a culture bag to rest on so that the culture bag is flatly placed on the bottom of the culture room 11.

The cover 20 is detachably disposed on the main body 10 to cover the opening so as to selectively close the culture room 11. Thereby, the environment in the culture room 11 becomes stable and controllable. Practically, corresponding buckle members are disposed on the periphery of the cover 20 and the periphery of the opening. When the cover 20 covers the opening of the main body 10, the buckle members are fastened to ensure the culture room 11 is independent from the exterior.

The connection seat 30 is adapted for being inserted into the receiving groove 12. The connection seat 30 is formed with at least one through hole 31 for at least one pipeline 90 to insert through so that the pipeline 90 connects the culture room 11 and the exterior therebetween. An end of the pipeline 90 is adapted for connecting to the culture bag. Therefore, the pipeline can be installed to the connection seat first, and the connection seat is inserted into the receiving groove. Comparing to the design that the pipeline is directly disposed on the main body to penetrate it, the pipeline and the connection seat can be a single module to facilitate installation.

The gas module 40 includes a carbon dioxide senser and a gas source connector. The carbon dioxide senser is arranged in the culture room 11 and is electrically connected to the gas source connector. The gas source connector is disposed on the main body 10 to connect to a gas source at the exterior so as to inject gas into the culture room 11. Preferably, the gas module 40 is disposed on a lateral side of the main body 10. In the present embodiment, the gas module 40 and the connection seat 30 are arranged at two opposite ends of the culture room 11.

The temperature module is disposed on one of the main body 10 and the cover 20, and includes a heater and a temperature detecting member 24. The temperature detecting member 24 is electrically connected to the heater. The temperature detecting member 24 is adapted for detecting the temperature in the culture room 11 and selectively driving the heater to heat up the culture room 11. Thereby, the temperature in the culture room 11 can be maintained at an appropriate amount which is near the body temperature of human. Practically, in the present embodiment, the heater is a heating glass 22. The cover 20 is formed with an observation opening 21 at the center thereof. The observation opening 21 has a size corresponding to the size of the heating glass 22. The heating glass 22 covers the observation opening 21. A pivotable cover 23 is further arranged at a side of the heating glass 22 opposite to the culture room 11. The pivotable cover 23 can selectively covers the heating glass 22 to prevent the light from entering the culture room 11 but also to allow the user to take a look into the culture room 11 by pivoting the pivotable cover 23. The temperature detecting member 24 can be disposed on the face of the cover 20 facing the culture room 11, but not limited to this. In other possible embodiments, the heater can be disposed on the bottom of the culture room to heat up the culture bag upward.

The illumination counting module includes a light source 51 and a light receiver 52. The light source 51 is arranged at one of the culture room 11 and the face of the cover 20 facing the culture room 11. The light receiver 52 is arranged at the other one of the culture room 11 and the face of the cover 20 facing the culture room 11. In the present embodiment, the light source 51 is disposed on the face of the cover 20 facing the culture room 11, and the light receiver 52 is arranged in the culture room 11. Thereby, the culture bag is located between the light source 51 and the light receiver 52 when it is placed in the culture room 11. The light source 51 is adapted for emitting light having the wavelength of 400-600 nm, 400-500 nm, or 400 nm toward the light receiver 52. The light penetrates the culture bag and is further received by the light receiver 52 so as to acquire the optical density of the fluid in the culture bag. Thereby, the cell density in the culture bag can be acquired too. Details are discussed below.

Specifically, the light source and the light receiver have to be arranged at positions that the light emitted by the light source is able to penetrate the culture bag. That is, the light source and the light receiver have to be arranged above and below the culture bag or the opposite. In addition, to avoid the light affects the growth of the most cells in the culture bag, the culture bag can be formed with an observation portion outward extending horizontally. The observation portion may be a protruding portion has a shape like finger or ear. The fluid and the cells in the culture bag are free to enter or leave the observation portion. The observation portion is located at a position corresponding to the positions of the light source and the light receiver so that the light penetrates only the observation portion. Therefore, other portions of the culture bag may not be affected by the light, or the effect to the cells can be reduced.

The culture bag positioning mechanism at least includes at least one slid track 61 and a clamping member 62. The slide track 61 is arranged at the culture room 11 and extends along a sliding direction. For example, the slide track can be directly disposed on the bottom of the culture room and connected to the clamping member. In other possible embodiments, the slide track can include the smooth surface of the bottom of the culture room and the magnetic components arranged under the culture room, and the clamping member is driven to move on the smooth surface of the bottom of the culture room by magnetic force. The clamping member 62 is slidably disposed on the slide track 61 to be adapted for pressing the culture bag so as to push the fluid in the culture bag toward one side of the culture bag. The clamping member 62 is slidable on the culture bag. In the present embodiment, two slide tracks 61 are provided. The two slide tracks 61 are arranged at two opposite sides of the culture room and extend parallel. Two ends of the clamping member 62 are slidably disposed on the two slide track 61 respectively and includes two horizontal rods spacedly arranged. The culture bag extends through the gap between the two horizontal rods and is clamped by the two horizontal rods. When the clamping member 62 slides along the two slide tracks 61, the two horizontal rods slide on the culture bag to push the fluid in the culture bag toward an end of the culture bag.

The culture bag positioning mechanism can further include at least one pulling mechanism 63. The pulling mechanism 63 is at least arranged at an end of the culture room and positions an end of the culture bag in a releasable way. Thereby, the culture bag can be pulled from a terminal end thereof, so that the deformation of the culture bag during the clamping member 62 moving can be prevented. Practically, the pulling mechanism 63 can be a clip to clamp the terminal end of the culture bag. However, the pulling mechanism can be a hook, a plug, or other similar objects, to correspond to the culture bags having hooks or suspension holes. In other possible embodiments, the pulling mechanism can include a positioning bolt and a magnetic piece adhered onto the positioning bolt. The culture bag can be clamped by the magnetic piece and the positioning bolt therebetween.

The ventilation rack 70 is detachably disposed on the bottom of the culture room 11 to support the culture bag. The ventilation rack 70 is formed with a plurality of hollow portions so as to facilitate the ventilation of the bottom of the culture bag. Therefore, the culture environments under and above the culture bag can be kept almost the same.

The culture fluid providing mechanism at least includes a cooling box 81 and a pump 82. The cooling box 81 is arranged in the main body 10 and is adapted for receiving at least one culture fluid container so as to maintain the culture fluid at low temperature. The pump 82 is also arranged in the main body 10. The at least one pipeline 90 is connected to the culture fluid container via the pump 82 so as to inject the cooled culture fluid into the culture bag. The pump 82 can be further connected to a control mechanism to automatically control the providing of the culture fluid.

As mentioned, the culture bag with the culture fluid and cells is arranged in the culture room and is further connected to the pipeline. In addition, the culture room is closed by the cover. Thus, automated cell culture can be achieved. Furthermore, the necessary observations and the measurement of the cell density can be proceeded by the sensors, the light receiver, and the calculations from the optical density. The data can be a reference for adjusting the providing of the culture fluid and the gas, and also the temperature in the culture room.

Specifically, the cell density is acquired by a linearity between the optical density (OD) and the cell density. To acquire the linearity, three classical cell strains are used in the experiments. In the experiments, the linearity is proved. Thus, the cell density can be easily acquired if the optical density is measured.

In the experiments below, 3*10^6 cells or 7 mL of cell fluid are used. It is centrifuged to give a precipitate and a supernatant liquid. 1mL of the supernatant liquid is used to give the cell density of 3*10^6. The cell fluid made from the supernatant liquid is serially diluted for 3 times to give 5 different cell densities of 3*10^6, 1*10^6, 3.3*10^5, 1.1*10^5, 3.7*10^4. 200 *µ*L of the diluted cell fluid is taken to inject into a 96-hole plate, and three samples are taken for each of the diluted cell densities. The supernatant liquid from the centrifuge step is taken for the control group, and three samples are taken wherein each of the sample is 200 *µ*L. The plate is scanned by the SpectraMax Paradigm Multi-Mode Microplate Reader in all wavelengths (230-1000 nm, 10 nm). The desirable wavelength is selected with the wavelength having a maximum difference of OD between the maximum density and the background. And then, plot the graph with OD and cell density.

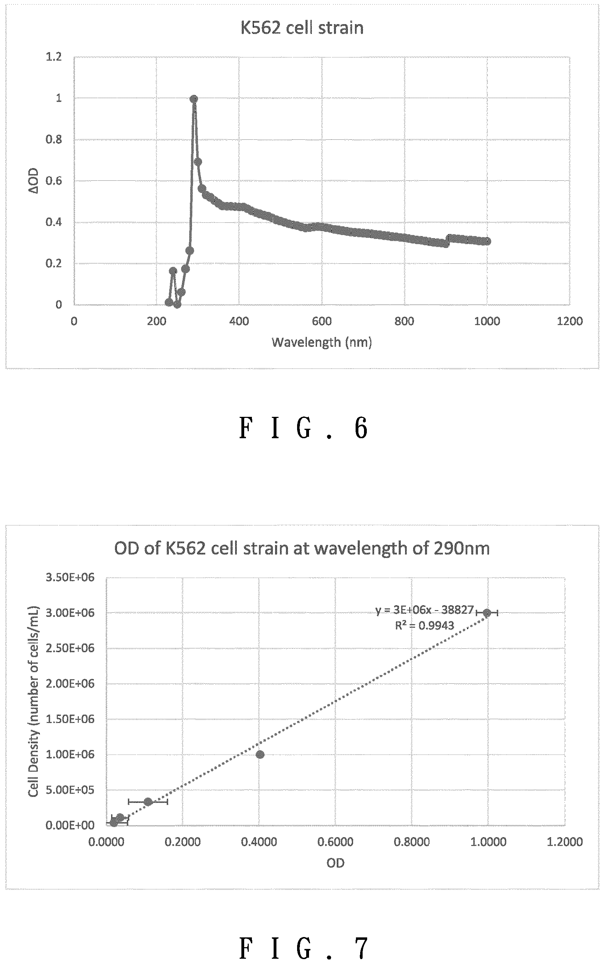

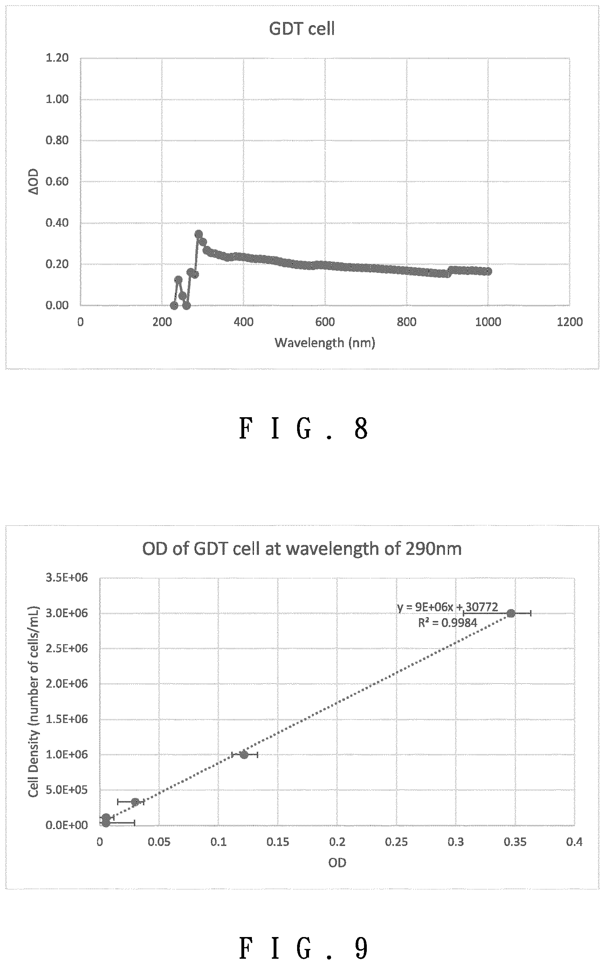

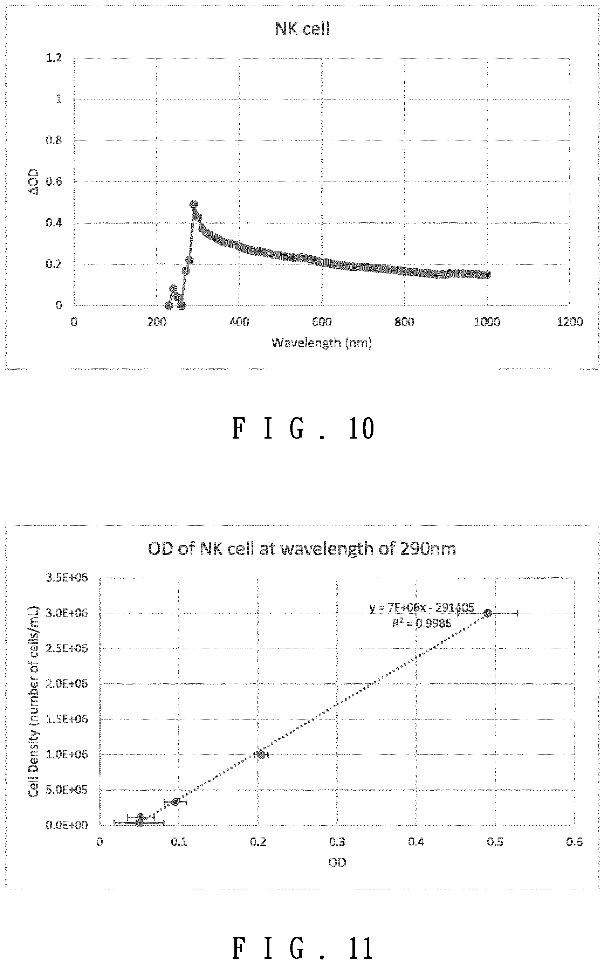

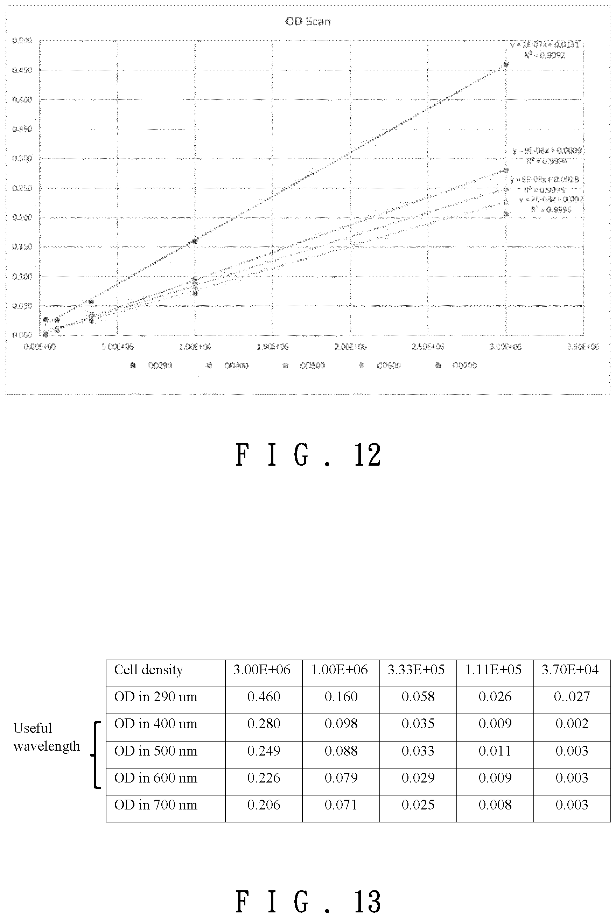

First, as shown in Fig. 6 and Fig. 7, the target of the experiment is K562 cell strain. As shown in Fig. 8 and Fig. 9, the target of the experiment is GDT (Gamma delta T) cells. As shown in Fig. 10 and Fig. 11, the target of the experiment is NK (Natural Killer) cells. In view of Fig. 6, Fig. 8, and Fig. 10, the three cells have the OD maximum at the wavelength of 290 nm, 400 nm, 500 nm, and 600 nm. These wavelengths are appropriate to measure the cell density. However, as shown in Fig. 12 and Fig. 13, a significant linearity is shown at the wavelength of 290 nm. Thus, the experiments are proceeded under the wavelength of 290 nm. Fig. 7, Fig. 9, and Fig. 11 show that the linearity between the OD and the cell density of each of the three cells exists indeed. Thus, it is reliable to acquire the cell density from the OD.

The experiments mentioned above are proceeded at the wavelength of 290 nm. However, the light of 290 nm has a higher energy so that the cells are possible to damage, or that the growth of the cell may be inhibited. Practically, the light having the wavelength of 400-600 nm, preferably 400-500 nm, more preferably 400 nm, is usually used for illumination counting. As shown in Fig. 12, even the light wavelength is 400-600 nm, the linearity between the OD and the cell density is still significant. However, the light having the wavelength of 400-600 nm has lower energy so that the cells may not be damaged. Specifically, the OD is significant when the light wavelength is 400 nm. Thus, the light having wavelength of 400 nm is more useful in the present invention.

As a result, the integrated automated cell culture device of the present invention is able to easily acquire the data of environment in the culture room and the cell density in the culture bag without opening the culture room and the culture bag for sampling. Thus, the data can be a standard to achieve the automated cell culture process. Thereby, the cost of culture can be reduced significantly. However, the environment of culture can be maintained stable, and the quality of the cells can be improved, which is desirable in the medical industry.

It will be apparent to those skilled in the art that various modifications and variations can be made to the structure of the present disclosure without departing from the scope or spirit of the disclosure. In view of the foregoing, it is intended that the present disclosure cover modifications and variations of this disclosure provided they fall within the scope of the following claims.

## Claims

1. An integrated automated cell culture device, including:
a main body, enclosing a culture room, having an opening on a top thereof which communicates the culture room, the main body further having a receiving groove at a lateral side thereof which communicates the culture room, a bottom of the culture room being adapted for a culture bag to place thereon;
a cover, detachably disposed on the main body to cover the opening in order to selectively close the culture room;
a connection seat, adapted for inserted into the receiving groove, the connection seat being formed with at least one through hole, the at least one through hole being adapted for at least one pipeline to insert through so that the at least one pipeline connects the culture room and the exterior, an end of the at least one pipeline toward the culture room being adapted for connecting to the culture bag;
a gas module, including a carbon dioxide senser and a gas source connecting member, the carbon dioxide sensor being arranged in the culture room and being electrically connected to the gas source connecting member, the gas source connecting member being disposed on the main body to be adapted for a gas source at the exterior to connect to so as to provide gas into the culture room;
a temperature module, disposed on at least one of the main body and the cover, including a heater and a temperature detecting member, the temperature detecting member being electrically connected to the heater, the temperature detecting member being adapted for detecting a temperature in the culture room and selectively driving the heater to heat up the culture room;
at least one slide track and a clamping member, the at least one slide track being arranged in or beside the culture room and extending along a sliding direction, the clamping member being slidably disposed on the at least one slide track and being adapted for pressing the culture bag in order to prohibit a fluid in the culture bag to flow to one side of the culture bag, the clamping member being slidable on the culture bag.

2. The integrated automated cell culture device of claim 1, further including an illumination counting module, the illumination counting module including a light source and a light receiver, the light source being disposed on one of the culture room and a face of the cover facing the culture room, the light receiver being disposed on the other one of the culture room and the face of the cover facing the culture room so that the culture bag is located between the light source and the light receiver when the culture bag is placed in the culture room, the light source being adapted for emitting light having a wavelength of 400-600 nm toward the light receiver, light emitted by the light source penetrating the culture bag and being further received by the light receiver so as to acquire an optical density (OD) of the fluid in the culture bag, thereby a cell density in the culture bag is obtained.

3. The integrated automated cell culture device of claim 2, further including said culture bag, the culture bag having at least one observation portion outward extending horizontally, the fluid and cells in the culture bag being free to enter or leaving the observation portion, positions of the light source and the light receiver corresponding to a position of the observation portion so that the light emitted by the light source penetrates the culture bag via the observation portion.

4. The integrated automated cell culture device of claim 2, wherein the light source being adapted for emitting light having the wavelength of 400 nm toward the light receiver.

5. The integrated automated cell culture device of claim 1, wherein the cover is formed with an observation opening at an intermediate portion of the cover, a heating glass is arranged on the observation opening to cover the observation opening, a pivotable cover is arranged at a side of the heating glass opposite to the culture room, the pivotable cover selectively covers the heating glass from outside, the heating glass is said heater, the temperature detecting member is disposed on the face of the cover facing the culture room.

6. The integrated automated cell culture device of claim 1, further including at least one pulling mechanism, the at least one pulling mechanism is at least arranged at an end of the culture room so as to clamp an end of the culture bag.

7. The integrated automated cell culture device of claim 1, wherein the gas module and the connection seat are arranged at two opposite ends of the culture room.

8. The integrated automated cell culture device of claim 1, further including a ventilation rack placed on the bottom of the culture room to support the culture bag.

9. The integrated automated cell culture device of claim 8, wherein the ventilation rack is formed with a plurality of hollow portions to facilitate ventilation of a bottom of the culture bag.

10. The integrated automated cell culture device of claim 1, further including a cooling box and a pump, the cooling box being arranged in the main body to receive at least one culture fluid container, the pump being also disposed on the main body, the at least one pipeline being connected to the at least one culture fluid container via the pump so as to provide fluid into the culture bag.
